# EUROPEAN PATENT SPECIFICATION

(11) **EP 2 023 909 B1**
(45) Date of publication and mention of the grant of the patent: **18.11.2009**
(21) Application number: 07728391.9
(22) Date of filing: 23.04.2007
(51) Int. Cl.: A61K 31/00, A61K 31/439, A61K 31/4439, A61K 31/551, A61P 25/18

(54) **MUSCARINIC AGONISTS TO TREAT IMPULSE CONTROL DISORDERS**
MUSCARIN-AGONISTEN ZUR BEHANDLUNG VON IMPULSKONTROLLSTÖRUNGEN
AGONISTES MUSCARINIQUES POUR TRAITER LES TROUBLES DU CONTROL DES PULSIONS

(30) Priority: 04.05.2006 EP 06113476; 04.05.2006 US 797355 P
(43) Date of publication of application: 18.02.2009
(73) Proprietor: Solvay Pharmaceuticals B.V., 1381 CP Weesp (NL)
(72) Inventor: DE BRUIN, Natasja M.W.J., 1383 CP Weesp (NL); VAN DRIMMELEN, Marlies L., 1381 CP Weesp (NL); HERREMANS, Arnoldus H.J., 1381 CP Weesp (NL); TULP, Martinus Th.M., 1381 CP Weesp (NL); KRUSE, Cornelis G., 1381 CP Weesp (NL)
(74) Representative: Verhage, Marinus
(86) International application number: PCT/EP2007/053934
(87) International publication number: WO 2007/128674

(56) References cited:
- EP-A- 0 525 879
- WO-A-2006/017614
- WO-A-2006/067494
- US-A1- 2004 116 505
- VARIOUS: "DIAGNOSTIC AND STATISTICAL MANual of merntal disorders" AMERICAN PSYCHIATRIC ASSOCIATION , ARLINGTON

## Description

### BACKGROUND OF THE INVENTION

An impulsive person is apt to be moved by a sudden impulse. This behaviour often is associated with a lack of self-control. Impulsivity therefore, has a substantial impact on individuals as well as on society. Impulse control disorders (ICD's) are characterized by failure to resist an impulse, drive or temptation to perform an act that is harmful to the person or to others. In most cases, the individual feels an increasing sense of tension or arousal before committing the act, and then experiences pleasure, gratification, or release at the time of committing the act. After the act is performed, there may or may not be regret or guilt.

Impulse control disorders are a separate group of psychiatric disorders, listed in the *"Diagnostic and Statistical Manual of Mental Disorders"* (DSM-IV) of the American Psychiatric Association as a residual category consisting of impulse control disorders 'Not Elsewhere Classified' (NEC) and impulse control disorders 'Not Otherwise Specified' (NOS). The first consist of: intermittent explosive disorder, pyromania, kleptomania, pathological gambling and trichotillomania. No specific disorders are mentioned in DSM-IV under the heading: 'impulse control disorders NOS', but this group is defined as "a category for disorders of impulse control that do not meet the criteria for any specific impulse control disorder or for any other mental disorder having features involving impulse control (such as borderline, antisocial, histrionic and narcissistic personality disorders)". In the scientific and patent literature a number of such impulse control disorders, also referred to as 'atypical impulse control disorders', are described, for instance: compulsive buying disorder, binge eating and binge drinking disorder, impulsive self-injurious behaviour such as pathological skin picking, nail-biting and nose-picking, gouging, head banging and self-biting; paraphilic sexual addictions, lack of control of a person's sexual impulses, include exhibitionism, fetishism, frotteurism, pedophilia, masochism, sadism, transvestic fetishism and voyeurism, as well as compulsive Internet use and excessive mobile phone use.

Patients suffering form impulse control disorder have been treated by psychotherapy, behaviour modification, hypnosis, relaxation therapy and administration of varied pharmaceutical preparations, the latter with little or no success. Historically, impulse control disorders have been considered refractory to known pharmacological or psychotherapeutic treatments. Therefore, a continuing need exists for agents thatt will be effective to treat the symptoms associated with ICD's, either by eliminating or by reducing them. In different patent and patent applications a variety of molecular mechanisms are claimed to be of therapeutic value in impulse control disorders: opioid antagonists (US 5,780,479), anticonvulsants (WO 02/43731); serotonin antagonists (US 2001023254); 5-HT_{1A} agonists (WO 94/13659), serotonin reuptake inhibitors (WO 92/18005) and cannabinoid antagonists (US 2004/ 0077650).
Muscarinic agonists have been claimed as treatment for cognitive impairment, psychosis, affective disorders, mania, and behavioural disorders (WO 2006/067494, WO 2006/017614 and EP 0525 879) as well as for the treatment of tics, tremors and related disorders (US 2004/116505). These disorders are quite distinct from impulse control disorders (DSM-IV)

### DETAILED DESCRIPTION OF THE INVENTION

The goal of the present invention is to develop drugs for the therapy of impulse control disorders, which have a mechanism of action different from that of drugs currently on the market, and which are likely to improve impulsivity control without negative effects on attention and concentration.

Surprisingly, muscarinic-1 agonists were found active in an animal model predictive of impulsive behavior in humans: the attenuation of MK801 induced increase of anticipatory responses of rats in the 5-Choice-Serial-Reaction-Time task, an effect associated with impulsive behavior (Cole, 1987; Ruotsalainen, 2000). Muscarinic agonists are active at doses in the range of 0.1 - 100 mg/kg after oral administration, and their unique pharmacological profile clearly indicates therapeutic potential in impulse control disorders.

In a preferred embodiment, the invention embraces the following muscarinic-1 agonists: AF-150, AF-151, alvameline, ACP-104, CDD-34, CDD-98, CDD-0097, CDD-0102, CDD-190, CDD-0199-J, CDD-0235-J, CDD-0304, cevimeline, CPR-2006, CS-932, desmethylclozapine, FPL-14995, FPL-15467, KAD-193R, L-680648, L-687306, L-689660, MCNa-343, milameline, nebracetam, NGX-267, PD-151832, sabcomeline, SDZ-210-086, SR-46559A, talsaclidine fumarate, tazomeline, xanomeline, YM-796 and YM-954.

Most preferred is the use of the muscarinic-1 agonist xanomeline

The compounds of the invention, as well as the pharmacologically acceptable salts thereof, have muscarinic-1 receptor agonistic activity. They are useful in treating impulse control disorders, including intermittent explosive disorder, pyromania, kleptomania, pathological gambling, trichotillomania, compulsive buying disorder, binge eating and binge drinking disorder, impulsive self-injurious behaviour such as pathological skin picking, nail-biting and nose-picking, gouging, head banging and self-biting; paraphilic sexual addictions, including exhibitionism, fetishism, frotteurism, pedophilia, masochism, sadism, transvestic fetishism and voyeurism; compulsive Internet use and excessive mobile phone use.

### DEFINITIONS

### DSM-IV: IMPULSE CONTROL DISORDERS NOT ELSEWHERE CLASSIFIED (NEC)

**Intermittent explosive disorder (IED)** is a disease characterized by the occurrence of discrete episodes of failure to resist aggressive impulses that result in serious assaultive acts or destruction of property. The degree of aggressiveness expressed during an episode is grossly out of proportion to any provocation or precipitating psychosocial stressor. The diagnosis IED is made only after other mental disorders that might account for episodes of aggressive behaviour have been ruled out (e.g. antisocial personality disorder, borderline personality disorder, a psychotic disorder, a manic episode, conduct disorder or ADHD. In IED, the aggressive episodes are not due to the direct physiological effects of a substance (e.g. an abused drug or medication) or a general medical condition (e.g. head trauma). Patients may describe the aggressive episodes as 'spells' of 'attacks' in which the explosive behaviour is preceded by a sense of tension or arousal and is followed immediately by a sense of relief. Later the individual may feel upset, remorseful, regretful or embarrassed about the aggressive behaviour.
**Pyromania**, also referred to as arsonism, pyrophilia or pathological fire-setting, is characterized by the presence of multiple episodes of deliberate and purposeful fire setting. Patients experience tension or affective arousal before setting a fire, and are characterized by fascination with, interest in, curiosity about, or attraction to fire and its situational contexts. Individuals with this disorder are often regular watchers at fires in their neighborhoods, may set off false alarms, and derive pleasure from institutions, equipment and personnel associated with fire. Pyromaniacs experience pleasure, gratification of a release of tension when setting a fire, witnessing its effects or participating in its aftermath. The fire setting is not done for monetary gain, as an expression of sociopolitical ideology, to conceal criminal activity, to express anger or vengeance, to improve one's living circumstances, or in response to a delusion or a hallucination, and the fire setting does not result from impaired judgement (e.g. dementia, mental retardation or substance abuse).
**Kleptomania** is characterized by a recurrent failure to resist impulses to steal items even though they are not needed for personal use or for their monetary value. The individual experiences a rising subjective sense of tension before the theft and feels pleasure, gratification or relief when committing the theft. The stealing is not committed to express anger or vengeance, is not done in response to a delusion or hallucination, and is not better counted for by conduct disorder, a manic episode or antisocial personality disorder. The objects are stolen despite the fact that are typically of little value to the individual who could have afforded to pay for them, and often gives them away or discards them. Although patients will generally avoid stealing when immediate arrest is probable, they usually do not preplan the thefts or fully take into account the chances of apprehension. The stealing is done without assistance from, or collaboration with, others.
**Pathological gambling**, also known as 'gambling disorder' or 'problem gambling', is characterized by a persistent and recurrent maladaptive gambling behaviour that disrupts personal, family or vocational pursuits. The individual may be preoccupied with gambling. Most patients say that they are seeking 'action' even more than money. Increasingly larger bets, or greater risks, may be needed to continue to produce the desired level of excitement. Gambling often continues despite repeated efforts to control, cut back, or stop the behaviour. The individual may gamble as a way of escaping from problems or to relieve a dysphoric mood. A pattern of 'chasing' one's losses may develop, with an urgent need to keep gambling to undo losses. Individuals may lie to family members, therapists or others to conceal the extend of involvement with gambling. When his borrowing resources are restrained, the person may resort to antisocial behaviour (fraud, theft) to obtain money. The individual may have jeopardized or lost a significant relationship, job or educational or career opportunity because of gambling.
**Trichotillomania**, pathological hair-pulling, is defined as 'recurrent failure to resist impulses to pull out one's hair, resulting in noticeable hair loss'. It is a common disorder characterized by plucking of hairs from head, eyelashes, eyebrows and other parts of the body. Trichotillomania is an often chronic and socially debilitating disorder.

### DSM-IV: IMPULSE CONTROL DISORDERS NOT OTHERWISE SPECIFIED (NOS)

**Compulsive buying disorder,** also referred to as compulsive shopping disorder, uncontrolled buying, is generally recognized as an impulse control disorder. It shares many characteristics with kleptomania.
**Binge eating disorder (BED)** is characterized by discrete periods of binge eating during which large amounts of food are consumed in a discrete period of time. A sense of control over eating is absent. Binge eating disorder is distinguished from Bulimia Nervosa by the absence of the regular use of inappropriate compensatory behaviors such as self-induced vomiting, misuse of laxatives and other medications, fasting and excessive exercise that are characteristic of the latter. **Binge drinking disorder** is - *mutatis mutandis -* the same as binge eating disorder.
**Impulsive self injurious behaviour** (also referred to as repetitive self-mutilation), is the inability of an individual to control the impulse to scratch, pick, lick or cause self-injury by repeated mechanical irritation of an injured area. This behaviour becomes manifest in different forms, for instance **pathological skin picking (PSP)**, a severe and chronic psychiatric and dermatologic problem which can lead to significant suffering, dysfunction and disfigurement e.g. in the form of facial skin lesions; **pathological nail-biting** (onychophagia); **pathological nose-picking** (rhinotillexo-mania); **gouging** (auto-enucleation): the use of one's fingers to stick out one's own eyes, a rare and severe form of orbital trauma; **head banging** and **self-biting.**
**Paraphilic sexual addictions** (erotomania, hypersexuality), lack of control of a person's sexual impulses, are characterized by recurrent, intense sexual urges, fantasies, or behaviors that involve unusual objects, activities, or situations and cause clinically significant distress or impairment in social, occupational, or other important areas of functioning. Paraphilias include **exhibitionism** (exposure of genitals), **fetishism** (use of non-living objects), **frotteurism** (touching and rubbing against a nonconsenting person), **pedophilia** (focus on prepubescent children), **sexual masochism** (receiving humiliation or suffering), **sexual sadism** (inflicting humiliation or suffering), **transvestic fetishism** (cross dressing) and **voyeurism** (observing sexual activity).
Spending many hours a day behind a computer screen, searching the Internet, may be the characteristic of a well paid job, it may also cause clinically significant distress or impairment in social, occupational, or other important areas of functioning, in particular when it is not job-related, and restricted to a person's leisure time. Already some clinicians have listed **'compulsive Internet use'** as impulse control disorder.
In the eyes of many adults most teenagers make excessive use of mobile phones. Most juveniles perceive this as quite normal. In certain individuals however, this behavior reaches pathological heights. It therefore may be anticipated that '**excessive mobile phone use**' will be recognized as impulse control disorder, and very well may prove not to be refractory to pharmacological treatment.

To provide a more concise description, some of the quantitative expressions given herein are not qualified with the term **"about".** It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.
Throughout the description and the claims of this specification, the word **"comprise"** and variations of the word, such as "comprising" and "comprises" is not intended to exclude other additives, components, integers or steps.
The term **"composition"** as used herein encompasses a product comprising specified ingredients in predetermined amounts or proportions, as well as any product that results, directly or indirectly, from combining specified ingredients in specified amounts. In relation to pharmaceutical compositions, this term encompasses a product comprising one or more active ingredients, and an optional carrier comprising inert ingredients, as well as any product that results, directly or indirectly, from combination, complexation or aggregation of any two or more of the ingredients, or from dissociation of one or more of the ingredients, or from other types of reactions or interactions of one or more of the ingredients. In general, pharmaceutical compositions are prepared by uniformly and intimately bringing the active ingredient into association with a liquid carrier or a finely divided solid carrier or both, and then, if necessary, shaping the product into the desired formulation. The pharmaceutical composition includes enough of the active object compound to produce the desired effect upon the progress or condition of diseases. Accordingly, the pharmaceutical compositions of the present invention encompass any composition made by admixing a compound of the present invention and a pharmaceutically acceptable carrier. By "**pharmaceutically acceptable"** it is meant the carrier, diluent or excipient must be compatible with the other ingredients of the formulation and not deleterious to the recipient thereof.
From the binding affinity measured for a muscarinic agonist, one can estimate a theoretical lowest effective **dose.** At a concentration of the compound equal to twice the measured Kᵢ-value, nearly 100% of the muscarinic receptors likely will be occupied by the compound. By converting that concentration to mg of compound per kg of patient one obtains a theoretical lowest effective dose, assuming ideal bioavailability. Pharmacokinetic, pharmaco-dynamic, and other considerations may alter the dose actually administered to a higher or lower value. The dose of the compound to be administered will depend on the relevant indication, the age, weight and sex of the patient and may be determined by a physician. The dosage will preferably be in the range of from 0.01 mg/kg to 10 mg/kg. The typical daily dose of the active ingredients varies within a wide range and will depend on various factors such as the relevant indication, the route of administration, the age, weight and sex of the patient and may be determined by a physician. In general, oral and parenteral dosages will be in the range of 0.1 to 1,000 mg per day of total active ingredients.
The term **"therapeutically effective amount"** as used herein refers to an amount of a therapeutic agent to treat or prevent a condition treatable by administrating a composition of the invention. That amount is the amount sufficient to exhibit a detectable therapeutic, preventative or ameliorative response in a tissue system, animal or human. The effect may include, for example, treating or preventing the conditions listed herein. The precise effective amount for a subject will depend upon the subject's size and health, the nature and extent of the condition being treated, recommendations of the treating physician (researcher, veterinarian, medical doctor or other clinician), and the therapeutics, or combination of therapeutics, selected for administration. Thus, it is not useful to specify an exact effective amount in advance. The term **"treatment"** as used herein refers to any treatment of a mammalian, preferably human condition or disease, and includes: (1) preventing the disease or condition from occurring in a subject predisposed to the disease, but not yet diagnosed as having it, (2) inhibiting the disease or condition, i.e., arresting its development, (3) relieving the disease or condition, i.e., causing the condition to regress, or (4) stopping the symptoms of the disease. As used herein, the term **"medical therapy"** intended s to include prophylactic, diagnostic and therapeutic regimens carried out *in vivo* or *ex vivo* on humans or other mammals. The term "subject" as used herein, refers to an animal, preferably a mammal, most preferably a human, who has been the object of treatment, observation or experiment.

### EXAMPLE 1: FORMULATIONS USED IN ANIMAL STUDIES

For subcutaneous (s.c.) administration: to the desired quantity (0.05mg/ml) of **MK801** in a glass tube, some glass beads were added and the substance was milled by vortexing for 20 sec. After addition of 2 ml of solvent (Saline 0.9%), the compound was dissolved by vortexing for 20 sec. The rest of the solvent minus 1 ml was added to the solution and vortexed for 20 sec. Then pH was measured (pH between 5 - 8) and vortexed (20 sec). The last volume was added to the solution, vortexed for 20 sec and a final pH check was done, the actual pH was notated.

For intraperitoneal *(i.p.)* administration: to the desired quantity (5 mg/ml) of **desmethylclozapine** in a glass tube, some glass beads were added and the substance was milled by vortexing for 20 sec. After addition of 2 ml of 1% methylcellulose and 5% mannitol in water, the compound was suspended by vortexing for 20 sec. The rest of the solvent minus 1 ml was added to the suspension and vortexed for 20 sec. Then pH was measured and set with 1 drop of 1.0 M NaOH (pH between 5 - 8) and vortexed (20 sec). The last volume was added to the suspension, vortexed for 20 sec and a final pH check was done, the actual pH was notated.

For intraperitoneal *(i.p.)* administration: to the desired quantity (5 mg/ml) of **xanomeline** in a glass tube, some glass beads were added and the substance was milled by vortexing for 20 sec. After addition of 2 ml of 1% methylcellulose and 5% mannitol in water, the compound was suspended by vortexing for 20 sec. and the tube was placed in an ultrasonic bath for 15 min. Before the rest of the solvent was added the suspension was vortexed for 20 sec. The rest of the solvent minus 1 ml was added to the suspension and vortexed for 20 sec. Then pH was measured and set with 5 drops of 1.0 M NaOH (pH between 5 - 8) and vortexed (20 sec). The last volume was added to the suspension, vortexed for 20 sec and a final pH check was done, the actual pH was notated.

### EXAMPLE 2: THE 5-CHOICE SERIAL REACTION TIME TASK PROTOCOL

### Animals

Male Wistar rats weighing 330 - 450 g (Harlan, The Netherlands) are housed in pairs under a 12-h light/dark cycle (lights off at 7:00 a.m.) and have daily access to a limited amount off food that keeps their body weight at approximately 85% of the free feeding weight. Water is available *ad libitum.* All procedures are in compliance with the European Communities Council Directive of November 24^{th} 1986 (86/609 EEC).

### Apparatus

Standard 5-CSRT boxes from MED associates Inc. Georgia, Vermont US are used. The curved wall of the chamber contains five 1.6 cm² holes, 2.2 cm deep, 2 cm above floor level. Each hole has an infra-red photocell beam crossing the entrance and illuminating a photo-electric cell for head entry detection. In every hole a yellow stimulus light can be presented. A food dispenser is located at the opposite wall. The box is illuminated by a 28 Volt 100 mA house light. The 5-CSRT boxes are placed in sound attenuating cubicles. On-line control of the apparatus and data collection are performed by a MED-PC experimental apparatus programming system (Tatham, 1989), running on a Dell Optiplex GX1 PC.

### Training

Rats are first trained to collect reinforcement pellets from the food dispenser and to collect reinforcements that are placed in the 5 holes in order to habituate the animals to the 5-CSRT task apparatus. Next, training of the 5-CSRT task starts. These sessions start by illumination of the light in one of the holes for 30 seconds (stimulus duration). A response by the rat into the illuminated hole or a response in that particular hole for a short period of time after the illumination (the limited hold 60 sec) is rewarded with the delivery of a food pellet, termination of the trial and a correct response is recorded. The light stimulus is presented in each of the holes 20 times during a session (total of 100 presentations), and the order of presentations is randomized. A response in any other hole (fault response) or a failure to respond at all during the stimulus presentation and the limited hold (omission) results in a 5 sec. period of darkness (time out) and termination of the trial. After termination of the trial a variable inter-trial interval is started (1, 3, 5, 7 or 9 sec) after which the next trial is started. Upon reaching a 75% correct performance level the duration of illumination of the light stimulus is reduced until a presentation of 0.5 sec (from 30 to 20, 10, 5, 2, 1, 0.9; 0.8; 0.7; 0.6; 0.5.) and a limited hold of 5 sec (in three steps from 30 to 20, 10 and 5 sec), is reached. (Robbins, 1993).

The following parameters are measured (Muir, 1996):
**Accuracy:** the percentage correct responses divided by the total responses made.
**Errors of omission:** the number of missed stimulus presentations.
**Correct responses:** the number of correct responses.
**Fault responses:** the number of responses in the wrong hole.
**Anticipatory responses:** the number of premature responses made preceding the presentation of the light stimulus.
**Persevering responses:** responses emitted after an incorrect or correct response.

### EXAMPLE 3: ACTIVITY IN THE 5-CHOICE SERIAL REACTION TIME TASK

MK801 was tested in a latin hexagon design, meaning that each rat received all test dosages (0, 0.01, 0.025 and 0.05 mg/kg *s.c*.) in a random order. The results of two different series of experiments are given in table 1:

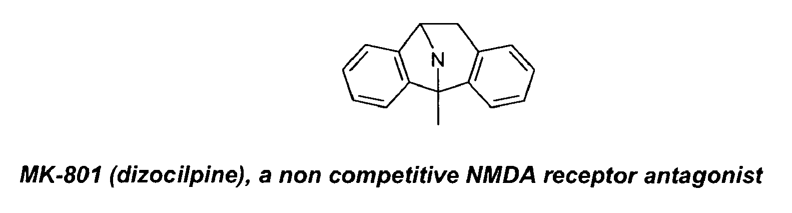

| **Table 1: RESPONSES IN THE 5-CHOICE SERIAL REACTION TIME TASK** | | | | |
|---|---|---|---|---|
| | | | | |

| **First series: numbers (%) of responses (± S.E.M.) at different dosages of MK801** | | | | |
|---|---|---|---|---|
| | | | | |
| dose of MK-801 (mg/kg, *s.c*.) | **0** | **0.01** | **0.025** | **0.05** |
| | | | | |
| percentage of correct responses | **72.8 ± 3.6** | **72.6 ± 2.8** | **70.0 ± 3.7** | **68.0 ± 3** |
| | | | | |
| number of correct responses | **49.8 ± 3.2** | **52.6 ± 3.3** | **53.8 ± 3.9** | **55.3 + 3.2** |
| number of fault responses | **18.8 ± 2.6** | **19.8 ± 2.2** | **22.6 ± 2.7** | **26.0 ± 2.7** |
| number of missed responses | **31.3 ± 3.1** | **27.7 ± 3.3** | **23.6** ± **2.9** | **18.7 ± 3.1 *** |
| number of anticipatory responses | **42.5 ± 6.8** | **51.3 ± 5.4** | **73.2 ± 8.7*** | **148.6 ± 38.6** * |
| persevering responses | **5.2 ± 0.9** | **7.9 ± 1** | **17.7 ± 4.1*** | **28.2 ± 4.7 *** |
| total number of responses | **68.7 ± 3.1** | **72.3 ± 3.3** | **76.4 ± 2.9** | **81.3 ± 3.1 *** |
| | | | | |

| **Second series: numbers (%) of responses (± S.E.M.) at different dosages of MK801** | | | | |
|---|---|---|---|---|
| | | | | |
| dose of MK-801 (mg/kg, *s.c*.) | **0** | **0.01** | **0.025** | **0.05** |
| | | | | |
| percentage of correct responses | **73.4 ± 2.2** | **73.8 ± 2.3** | **74.0 ± 2.5** | **72.7 ± 2.6** |
| | | | | |
| number of correct responses | **56.0 ± 3.3** | **60.8 ± 2.1** | **62.7 ± 2.9** | **62.3 ± 2.9** |
| number of fault responses | **19.8 ± 1.4** | **21.8 ± 2.1** | **21.8 ± 1.9** | **23.3 ± 2.3** |
| number of missed responses | **24.3 ± 2.8** | **17.5 ± 2.1** | **15.6 ± 1.9 *** | **14.5 ± 1.8 *** |
| number of anticipatory responses | **67.5 ± 13.1** | **78.1 ± 14.8** | **97.3 ± 16.2** | **148.3 ± 29.8*** |
| persevering responses | **6.9 ± 2.0** | **11.1 ± 3.1** | **10.7 ± 1.7** | **18.8 ± 4.2 *** |
| total number of responses | **75.8 ± 2.7** | **82.5 ± 2.1** | **84.4 ± 1.9 *** | **85.5 ± 1.8 *** |

From the data in table 1 it is evident that MK801 does not influence the numbers of correct responses and fault responses. The significant effects are on the number of anticipatory responses, persevering responses, missed responses and total number of responses: those are boosted except for missed responses which are decreased, an effect evident at 0.025 mg/kg and 0.05 mg/kg, and highly significant (* = p<0.05). This indicates that the animals show impulsive behavior.

**Xanomeline**, a mixed muscarine-M₁ and muscarine-M₄ agonist, and **desmethylclozapine,** a muscarinic-M₁ agonist and muscarinic-M₄ antagonist , were each tested in a latin hexagon design, meaning that each rat received all test-dosages (0, 1, 3 and 10 mg/kg*i*.*p*.) in a random order. The results are given in table 2:

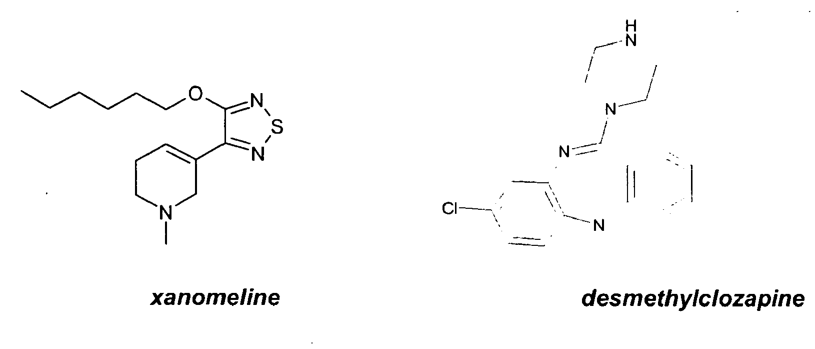

| **Table 2: RESPONSES IN THE 5-CHOICE SERIAL REACTION TIME TASK** | | | | |
|---|---|---|---|---|
| | | | | |
| **Numbers (%) of responses (± S.E.M.) at different dosages of Xanomeline** | | | | |
| | | | | |
| **dose of xanomeline** | **0 mg/kg** | **1 mg/kg** | **3 mg/kg** | **10 mg/kg** |
| | | | | |
| percentage of correct responses | **70.8 ± 2.3** | **70.0 ± 2.1** | **70.2 ± 2.5** | **72.1 ± 2.3** |
| | | | | |
| number of correct responses | **54.2 ± 2.5** | **53.0 ± 2.4** | **51.2 ± 2.5** | **37.2 ± 2.6 *** |
| number of fault responses | **22.2 ± 1.8** | **22.7 ± 1.6** | **21.7 ± 1.8** | 1**5.0 ± 1.8 *** |
| number of missed responses | **23.6 ± 2.1** | **24.3 ± 2.4** | **27.2 ± 2.2** | **46.8 ± 3.7 *** |
| number of anticipatory responses | **60.7 ± 7.9** | **58.0 ± 7.9** | **60.8 ± 4.9** | **21.5 ± 6.4 *** |
| persevering responses | **7.8 ± 1.3** | **6.3 ± 1.1** | **6.7 ± 1.4** | **4.9 ± 0.9** |
| total number of responses | **76.4 ± 2.1** | **75.7 ± 2.4** | **72.8 ± 2.2 53.2** | **± 3.7 *** |
| | | | | |

| **Numbers (%) of responses (± S.E.M.) at different dosages of Desmethylclozapine** | | | | |
|---|---|---|---|---|
| | | | | |
| **dose of desmethylclozapine** | **0 mg/kg** | **1 mg/kg** | **3 mg/kg** | **10 mg/kg** |
| | | | | |
| percentage of correct responses | **72.6 ± 2.7** | **72.9 ± 3.1** | **71.9 ± 3.2** | **72.6 ± 3.6** |
| | | | | |
| number of correct responses | **51.8 ± 2.7** | **52.2 ± 3.2** | **51.9 ± 3.1** | **48.5 ± 3.9** |
| number of fault responses | **19.4 ± 2.1** | **19.4 ± 2.2** | **20.1 ± 2.2** | **17.5 ± 2.0** |
| number of missed responses | **28.8 ± 2.4** | **28.4 ± 3.2** | **28.0 ± 2.2** | **34.0 ± 2.9** |
| number of anticipatory responses | **40.3 ± 4.2** | **50.2 ± 4.9** | **51.3 ± 7.5** | **34.5 ± 3.9** * |
| persevering responses | **7.6 ± 2.1** | **7.8 ± 1.4** | **8.8 ± 1.2** | **5.5 ± 0.9** |
| total number of responses | **71.2 ± 2.4** | **71.6 ± 3.2** | **72.0 ± 2.2** | **66.0 ± 2.9** |

The data given in table 2 show that at 10 mg/kg xanomeline and desmethylclozapine reduce the number of anticipatory responses: An effect associated with impulsive behavior (Cole, 1987; Ruotsalainen, 2000).

### INTERACTION STUDIES

Both xanomeline and desmethylclozapine were each tested in the same dose range against 0.05 mg/kg (s.c.) MK801, a dose shown to elicit unequivocal impulsive behavior (see Table 1). The results are given in table 3:

| **Table 3: RESPONSES IN THE 5-CHOICE SERIAL REACTION TIME TASK** | | | | | |
|---|---|---|---|---|---|
| | | | | | |

| **Numbers (%) of responses (± SEM) at different dosages of xanomeline against MK801** | | | | | |
|---|---|---|---|---|---|
| | | | | | |
| **dose of xanomeline** | **placebo** | **0 mg/kg** | **1 mg/kg** | **3 mg/kg** | **10 mg/kg** |
| | | | | | |
| % correct resp | **71.2 ± 2.6** | **66.9 ± 3.5** | **70.2 ± 3.8** | **71.6 ± 2.4** | **74.4 ± 2.8** |
| | | | | | |
| nr of correct resp | **51.1 ± 3.2** | **53.3 ± 3.9** | **58.7 ± 4.4** | **57.4 ± 2.7** | **50.9 ± 2.4** |
| nr of fault resp | **20.2 ± 1.6** | **25.9 ± 2.7** | **23.8 ± 2.4** | **22.7 ± 1.9** | **18.0 ± 2.2** |
| nr of missed resp | **28.8 ± 3.0** | **20.8 ± 3.4** | **17.6 ± 3.1** | **19.9 ± 2.4** | **31.1 ± 3.0** |
| nr anticipatory resp | **40.7 ± 3.8** | **133.8 ± 23.5** | **75.3 ± 10.5** | **99.3 ± 1.1** | **47.5 ± 8.4 *** |
| persevering resp | **6.5 ± 1.7** | **16.9 ± 2.6** | **15.3 ± 2.5** | **14.1 ± 3.5** | **8.5 ± 1.1** |
| total nr of resp | **71.3 ± 3.0** | **79.3 ± 3.4** | **82.4 ± 3.1** | **80.1 ± 2.4** | **68.9 ± 3.0** |
| | | | | | |

| **Numbers (%) of responses (± SEM) at different doses of desmethylclozapine vs MK801** | | | | | |
|---|---|---|---|---|---|
| | | | | | |
| desmethylclozapine | **placebo** | **0 mg/kg** | **1 mg/kg** | **3 mg/kg** | **10 mg/kg** |
| | | | | | |
| % correct responses | **74.3 ± 3.3** | **66.2 ± 3.8** | **67.2 ± 2.6** | **69.6 ± 2.6** | **70.1 ± 3.1** |
| nr of correct resp | **52.8 ± 3.1** | **53.8 ± 4.4** | **52.5 ± 3.4** | **55.2 ± 2.8** | **52.7 ± 3.9** |
| nr of fault resp | **18.2 ± 2.3** | **25.9 ± 2.1** | **25.1 ± 1.9** | **24.4 ± 2.4** | **21.6 ± 2.0** |
| nr of missed resp | **29.0 ± 2.7** | **20.3 ± 3.7** | **22.4 ± 3.1** | **20.4 ± 3.6** | **25.8 ± 3.7** |
| nr anticipatory resp | **46.9 ± 4.9** | **116.8 ± 19.9** | **141.8 ± 20.8** | **104.3 ± 18.4** | **78.6 ± 14.5 *** |
| persevering resp | **6.3 ± 1.4** | **20.2 ± 3.2** | **24.8 ± 2.8** | **16.3 ± 2.1** | **17.3 ± 3.4** |
| total nr of resp | **71.0 ± 2.7** | **79.7 ± 3.7** | **77.6 ± 3.1** | **79.6 ± 3.6** | **74.3 ± 3.7** |

The data given in table 3 clearly indicate that both xanomeline and desmethylclozapine antagonize the increase in anticipatory and persevering responses induced by MK801.

### REFERENCES, CITED PATENTS AND PATENT APPLICATIONS

Cole, B.J. and Robbins T.W., Psychopharmacology, 91, 458-466, 1987
Muir J.L. et al., Cerebral Cortex (6) 470-481, 1996.
Robbins, T.W., Muir, J.L., Killcross, A.S. and Pretsell, D.: "Methods for assessing attention and stimulus control in the rat". In Behavioural Neuroscience: A Practical Approach, Shagal, A., Oxfort University press, New York, Vol. 1, pp. 13-46, 1993.
0 Ruotsalainen et al., Psychopharmacology, 148, 111-123, 2000.
Tatham, T.A. and Zurn, K.R. "The MED-PC experimental apparatus programming system", Behavioural Research Methodes. Instrumental Computers. 21, 294-302, 1989
EP 0525 879 US 5,780,479; US 2001/023254; US 2004/0077650 and US 2004/0116505 WO 92/018005; WO 94/013659; WO 02/043731; WO 2006/017614 and WO 2006/067494

## Claims

1. Use of a muscarinic-1 (M₁) agonist for the preparation of a pharmaceutical composition for the treatment, amelioration or prevention of impulse control disorders.

2. Use as claimed in claim 1, wherein said muscarinic-1 (M₁) agonist is selected from: AF-150, AF-151, alvameline, ACP-104, CDD-34, CDD-98, CDD-0097, CDD-0102, CDD-190, CDD-0199-J, CDD-0235-J, CDD-0304, cevimeline, CPR-2006, CS-932, desmethylclozapine, FPL-14995, FPL-15467, KAD-193R, L-680648, L-687306, L-689660, MCNa-343, milameline, nebracetam, NGX-267, PD-151832, sabcomeline, SDZ-210-086, SR-46559A, talsaclidine fumarat, tazomeline, xanomeline, YM-796 and YM-954.

3. Use as claimed in claim 2, wherein said muscarinic-1 (M₁) agonist is xanomeline.

4. Use as claimed in any of the claims 1-3, wherein said impulse control disorders are impulse control disorders Not Elsewhere Classified.

5. Use as claimed in claim 4, wherein said impulse control disorders Not Elsewhere Classified are: intermittent explosive disorder, pyromania, kleptomania, pathological gambling and trichotillomania.

6. Use as claimed in any of the claims 1-3, wherein said impulse control disorders are impulse control disorders Not Otherwise Specified

7. Use as claimed in claim 6, **characterized in that** said impulse control disorders Not . Otherwise Specified are: compulsive buying disorder, binge eating and binge drinking disorder, impulsive self-injurious behaviour, including pathological skin picking, nail-biting and hose-picking, gouging, head banging and self-biting; paraphilic sexual addictions, including exhibitionism, fetishism, frotteurism, pedophilia, masochism, sadism, transvestic fetishism and voyeurism; compulsive internet use and excessive mobile phone use.

## Patentansprüche

1. Verwendung eines Muscarin-1(M₁)-Agonisten zur Herstellung einer pharmazeutischen Zusammensetzung zur Behandlung, Linderung oder Prävention von Impulskontrollstörungen.

2. Verwendung nach Anspruch 1, wobei der Muscarin-1(M₁)-Agonist ausgewählt ist aus: AF-150, AF-151, Alvamelin, ACP-104, CDD-34, CDD-98, CDD-0097, CDD-0102, CDD-190, CDD-0199-J, CDD-0235-J, CDD-0304, Cevimelin, CPR-2006, CS-932, Desmythylclozapin, FPL-14995, FPL-15467, KAD-193R, L-680648, L-687306, L-689660, MCNa-343, Milamelin, Nebracetam, NGX-267, PD-151832, Sabcomelin, SDZ-210-086, SR-46559A, Talsaclidin-Fumarat, Tazomelin, Xanomelin, YM-796 und YM-954.

3. Verwendung nach Anspruch 2, wobei der Muscarin-1(M₁)-Agonist Xanomelin ist.

4. Verwendung nach einem der Ansprüche 1 bis 3, wobei die Impulskontrollstörungen "nicht anderweitig klassifizierte" Impulskontrollstörungen sind.

5. Verwendung nach Anspruch 4, wobei die "nicht anderweitig klassifizierten" Impulskontrollstörungen sind: intermittierende explosive Störung, Pyromanie, Kleptomanie, pathologisches Glücksspiel und Trichotillomanie.

6. Verwendung nach einem der Ansprüche 1 bis 3, wobei die Impulskontrollstörungen "nicht anderweitig klassifizierte" Impulskontrollstörungen sind.

7. Verwendung nach Anspruch 6, **dadurch gekennzeichnet, dass** die "nicht anderweitig klassifizierten" Impulskontrollstörungen sind: zwanghafte Kaufstörung, Fress- und Trinkanfallstörung, impulsives selbstverletzendes Verhalten, einschließlich pathologisches Hautrupfen, Nägelkauen und Nasenbohren, Augenausdrücken ("gouging"), Kopfnicken und Selbstbeißung; paraphilische sexuelle Abhängigkeiten, einschließlich Exhibitionismus, Fetischismus, Frotteurismus, Pädophilie, Masochismus, Sadismus, transvestitischer Fetischismus und Voyeurismus; zwanghafte Internetnutzung und exzessive Handynutzung.

## Revendications

1. Utilisation d'un agoniste muscarinique-1 (M₁) pour la préparation d'une composition pharmaceutique destinée au traitement, à l'amélioration ou à la prévention des troubles du contrôle des impulsions.

2. Utilisation selon la revendication 1, où ledit agoniste muscarinique-1 (M₁) est sélectionné parmi : AF-150, AF-151, alvaméline, ACP-104, CDD-34, CDD-98, CDD-0097, CDD-0102, CDD-190, CDD-0199-J, CDD-0235-J, CDD-0304, céviméline, CPR-2006, CS-932, desméthyl-clozapine, FPL-14995, FPL-15467, KAD-193R, L-680648, L-687306, L-689660, MCNa-343, milaméline, nébracétam, NGX-267, PD-151832, sabcoméline, SDZ-210-086, SR-46559A, fumarate de talsaclidine, tazoméline, xanoméline, YM-796 et YM-954.

3. Utilisation selon la revendication 2, où ledit agoniste muscarinique-1 (M₁) est la xanoméline.

4. Utilisation selon l'une quelconque des revendications 1 à 3, où lesdits troubles du contrôle des impulsions sont des troubles du contrôle des impulsions sans autre classification.

5. Utilisation selon la revendication 4, où lesdits troubles du contrôle des impulsions sans autre classification sont le trouble explosif intermittent, la pyromanie, la kleptomanie, le jeu pathologique et la trichotillomanie.

6. Utilisation selon l'une quelconque des revendications 1 à 3, où lesdits troubles du contrôle des impulsions sont des troubles du contrôle des impulsions sans autre indication.

7. Utilisation selon la revendication 6, **caractérisée en ce que** lesdits troubles du contrôle des impulsions sans autre indication sont : le trouble des achats compulsifs, le trouble de la frénésie alimentaire (binge eating) et de la « biture expresse » (binge drinking), le comportement impulsif auto-délétère, y compris la dermatillomanie, l'onychophagie et la rhinotillexomanie, la duperie, la krouomanie et l'autodacnomanie ; les addictions sexuelles paraphiliques, y compris l'exhibitionisme, le fétichisme, le frotteurisme, la pédophilie, le masochisme, le sadisme, le fétichisme transvesti et le voyeurisme ; l'utilisation compulsive d'Internet et l'utilisation excessive des téléphones mobiles.
